(19)

**Europäisches Patentamt**

**European Patent Office**

**Office européen des brevets**

(11) **EP 4 235 143 A1**

(12) **EUROPEAN PATENT APPLICATION**

(43) Date of publication:
**30.08.2023 Bulletin 2023/35**

(21) Application number: **23158499.6**

(22) Date of filing: **24.02.2023**

(51) International Patent Classification (IPC):
**G01N 1/30** *(2006.01)* **G01N 1/31** *(2006.01)*

(52) Cooperative Patent Classification (CPC):
**G01N 35/0099; G01N 35/026; G01N 35/04;**
G01N 2035/00495

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **28.02.2022 JP 2022030373**
**28.02.2022 JP 2022030375**

(71) Applicant: **SYSMEX CORPORATION**
**Kobe-shi**
**Hyogo 651-0073 (JP)**

(72) Inventors:
• **Atarashi, Kazuyuki**
**Kobe-shi, Hyogo, 651-0073 (JP)**
• **Marliza, Madung**
**Kobe-shi, Hyogo, 651-0073 (JP)**
• **Sasaki, Yuto**
**Kobe-shi, Hyogo, 651-0073 (JP)**
• **Shohmi, Keiichiro**
**Kobe-shi, Hyogo, 651-0073 (JP)**
• **Yamaguchi, Miyu**
**Kobe-shi, Hyogo, 651-0073 (JP)**

(74) Representative: **Hoffmann Eitle**
**Patent- und Rechtsanwälte PartmbB**
**Arabellastraße 30**
**81925 München (DE)**

(54) **METHOD OF PREPARING A MEASUREMENT SPECIMEN AND SPECIMEN PREPARATION APPARATUS**

(57) A method is disclosed for preparing a measurement specimen to prepare a specimen from a blood sample that is easy to automate and has little effect on a signal of a cell to be measured. The method of preparing a measurement specimen includes removing red blood cells from a blood sample using a solid phase substance capable of binding to red blood cells and reacting the blood sample from which the red blood cell is removed with a reagent to immunostain cells.

EP 4 235 143 A1

**Description**

TECHNICAL FIELD

This invention relates to a method of preparing a measurement specimen to prepare a specimen from a blood sample and a specimen preparation apparatus.

BACKGROUND ART

An analysis using a flow cytometer is widely used to analyze leukocytes contained in blood. For example, a cell surface antigen and an intracellular antigen of cells to be measured contained in blood is immunostained by a labeled antibody, and the immunostained cells are detected by an optical measurement using a flow cytometer. When preparing a specimen for such flow cytometer analysis from a blood sample, it is necessary to remove red blood cells from the blood. Patent Document 1 discloses to collect a PBMC (peripheral blood mononuclear cell) from blood by density gradient centrifugation, stains a cell surface antigen and an intracellular antigen of the collected PBMC, and measures the stained specimen by a flow cytometer to identify a regulatory T cell. Patent Document 2 discloses a method of preparing a specimen for analysis by a flow cytometer. Patent Document 2 discloses a process for staining a blood cell, a process for lysing red blood cells with a lysing agent, and a process for fixation the stained blood cell.

[Patent Document 1] Japanese Patent Publication No. 2014-528697
[Patent Document 2] Japanese Patent No. 6170511

SUMMARY

The method of collecting a PBMC by centrifugation as disclosed in Patent Document 1 is commonly used as a method of removing red blood cells; however, an operation of removing only a layer of PBMC that forms in the middle of a centrifuge tube after centrifugation requires a manual technique of inserting a pipette tip to the layer of PBMC and pipetting only PBMC to avoid aspirating layers other than PBMC, making automation difficult. In addition, the method using a lysing agent in Patent Document 2 is easy to automate, but the lysing agent may directly or indirectly affect the amount of protein expression, and a signal obtained from the cell may change. Therefore, for example, the detection performance of a rare cell such as a regulatory T cell disclosed in Patent Document 1 may be reduced. Therefore, there is a need for a method of preparing a measurement specimen and a specimen preparation apparatus for preparing a specimen from a blood sample that is easy to automate and has little effect on the signal (fluctuation of a measurement result).

This invention is directed to the realization of a method of preparing a measurement specimen and a specimen preparation apparatus for preparing a specimen from a blood sample that is easy to automate and has little influence on a signal of cells to be measured.

In order to achieve the above-mentioned purpose, a method of preparing a measurement specimen according to a first invention includes: removing red blood cells from a blood sample using a solid phase substance capable of binding to red blood cells; and reacting the blood sample from which the red blood cell is removed with a reagent for immunostaining cells.

The method of preparing a measurement specimen according to the first invention removes red blood cells from a blood sample using a solid phase substance capable of binding to red blood cells, as described above. In this way, the red blood cell can be removed with the solid phase substance bound to the red blood cell, thereby facilitating automation of a process of removing the red blood cell. In addition, unlike a case where red blood cells are removed by dissolving the red blood cell with a lysing agent, the effect on a signal of a target cell to be measured (fluctuation of a measurement result) can be reduced.

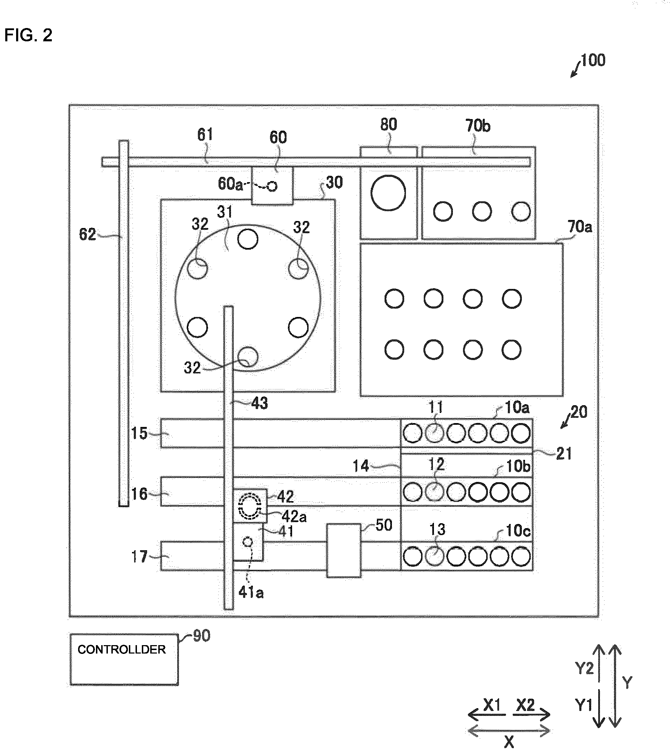

A specimen preparation apparatus according to a second invention is, as illustrated in FIG. 2, a specimen preparation apparatus (100) for preparing a measurement specimen by reacting a blood sample with a reagent, and includes a first processing section (20) that removes red blood cells from a blood sample using a solid phase substance capable of binding to red blood cells and a second processing section (30) that reacts the blood sample from which the red blood cell is removed with a reagent for immunostaining cells.

As described above, the specimen preparation apparatus according to the second invention includes the first processing section (20) that removes red blood cells from a blood sample using a solid phase substance capable of binding to red blood cells. In this way, the red blood cell can be removed with the solid phase substance bound to the red blood cell, thereby facilitating automation of a process of removing the red blood cell. In addition, unlike the case where red blood cells are removed by dissolving the red blood cell with a lysing agent, the effect on a signal of a target cell to be measured (fluctuation of a measurement result) can be reduced.

According to the present invention, it is possible to realize a method of preparing a measurement specimen and a specimen preparation apparatus which are easy to automate and have less influence on the signal of a target cell to be measured.

BRIEF DESCRIPTION OF DRAWINGS

FIG. 1 is a diagram illustrating an overview of a first embodiment to a third embodiment of removing red blood cells from a blood sample using a specimen preparation apparatus.

FIG. 2 is a schematic diagram illustrating an overview of a configuration example of the specimen preparation apparatus.

FIG. 3 is a diagram illustrating an overview of a processing flow by the specimen preparation apparatus.

FIG. 4 is a diagram illustrating a specific example of a specimen preparation process.

FIGS. 5A, 5B, and 5C are diagrams illustrating a movement of a rack when transferring a reaction container by the specimen preparation apparatus.

FIGS. 6A, 6B, 6C, and 6D are diagrams illustrating a movement of a rack when dispensing a sample by the specimen preparation apparatus.

FIGS. 7A, 7B, 7C, 7D, and 7E are diagrams illustrating a movement of a rack during processing by a first processing section of the specimen preparation apparatus.

FIGS. 8A, 8B, 8C, and 8D are diagrams illustrating a movement of a rack after processing by the first processing section of the specimen preparation apparatus.

FIG. 9 is a diagram illustrating a detection result of Teff cells in an embodiment and a comparative example.

FIG. 10 is a diagram illustrating a detection result of Treg cells in an embodiment and a comparative example.

FIG. 11 is a scattergram illustrating fractionated T cells with a high expression level of CD4 among cells fractionated into lymphocytes in an embodiment.

FIG. 12 is a scattergram illustrating fractionated T cells with a high expression level of CD4 among cells fractionated into lymphocytes in a comparative example.

FIG. 13 is a histogram illustrating fractionated cells with a low expression level of CD62L (CD4+/CD62L$^{Low}$ T cell) among CD4+T cells in an embodiment.

FIG. 14 is a histogram illustrating fractionated cells with a low expression level of CD62L (CD4+/CD62L$^{Low}$ T cell) among CD4+T cells in a comparative example.

FIG. 15 is a scattergram illustrating fractionated T cells with high expression levels of CD25 and FOXP3 (CD4+/CD25+/FOXP3+T cell) among CD4+T cells in an embodiment.

FIG. 16 is a scattergram illustrating fractionated T cells with high expression levels of CD25 and FOXP3 (CD4+/CD25+/FOXP3+T cell) among CD4+T cells in a comparative example.

## DETAILED DESCRIPTION

A method of preparing a measurement specimen of the present embodiment (hereinafter also referred to as "a preparation method of the embodiment") includes removing red blood cells from a blood sample using a solid phase substance capable of binding to red blood cells and reacting the blood sample from which red blood cells are removed with a reagent for immunostaining cells. According to the preparation method of the embodiment, a measurement specimen is prepared in which red blood cells are removed and a specific cell is immunostained.

A measurement specimen prepared by the method of the embodiment is a measurement specimen in which cells contained in a blood sample are immunostained, and is a measurement specimen particularly suitable for a measurement by a flow cytometer. The blood sample is, for example, a sample of biological origin, for example, whole blood collected from a subject. The subject is primarily human but may be other animals other than humans.

The blood sample includes at least cells to be measured and red blood cells. The cell to be measured is, for example, a white blood cell, more specifically a lymphocyte. More specifically, the cell is a T cell. More specifically, the cell to be measured is a regulatory T cell (hereafter referred to as a Treg cell) and an effector T cell (hereafter referred to as a Teff cell) among T cells. These Treg cell and Teff cell are distinguished from each other and counted by immunostaining with cells surface antigen and an intracellular antigen specifically expressed on Treg cell and Teff cell and by obtaining a fluorescent signal obtained by a flow cytometry, as described below.

A signal emitted from cells other than Treg cell and Teff cell is noise that inhibits accurate classification and counting. The noise from the red blood cell, which is included in large quantities in a blood sample, is particularly large, so the red blood cell needs to be removed before a measurement by the flow cytometry.

Conventionally, a lysing agent is widely used to remove red blood cells contained in a blood sample. For example, an ammonium chloride and a surfactant are commonly used as lysing agents. These lysing agents are inexpensive and easy to prepare in that the red blood cell can be removed by simply mixing with a blood sample; therefore, these lysing agents are widely used in a preparation of a measurement specimen to measure a common antigen marker, such as CD4 and CD25. However, inventors examined that in the measurement of specific cell surface antigen and intracellular antigen, a method of removing red blood cells using a conventional lysing agent affects a fluorescent signal in the measurement of cells to be measured, especially Treg cell and Teff cell in an embodiment described below. Also, further research by the inventors reveal that instead of using a lysing agent to remove red blood cells, a solid phase substance that can bind to red blood cells can be used to remove red blood cells, thereby reducing the effect on the fluorescent signal. A method of preparing a measurement specimen according to the present embodiment is described in detail below.

<Red blood cell removal>

A solid phase substance capable of binding to red blood cells is, for example, a solid phase substance capable of capturing and sticking red blood cells floating in a blood sample by contacting the blood sample. The solid phase substance capable of binding to red blood cells binds specifically to red blood cells and not to cells to be measured, e.g., a white blood cell. To bind specifically to red blood cells, an antibody capable of binding to red blood cells is preferably fixed on a surface of the solid phase substance. The antibody capable of binding to red blood cells is preferably, for example, an antibody capable of binding to a surface antigen of the red blood cell by antigen-antibody reaction (hereinafter referred to as red blood cells capture antibody), such as a CD235a antibody, CD55 antibody, etc. CD235a antibody is particularly preferable as an antibody capable of binding specifically to red blood cells. In the case of removing red blood cells in blood of mouse origin, TER-119 antibody may also be used.

As a method of fixation the red blood cell capture antibody on a surface of a solid phase substance, for example, the interaction of avidin or streptavidin with biotin is preferable. For example, by reacting a biotinylated red blood cell capture antibody with a solid phase substance to which streptavidin is bound, the red blood cell capture antibody can be fixed on the surface of the solid phase substance. The red blood cell capture antibody may be mixed with a blood sample with the red blood cell capture antibody pre-fixed on the solid phase substance, or the red blood cell capture antibody and the solid phase substance may be mixed separately with the blood sample and the red blood cell capture antibody is fixed on the solid phase substance in the blood sample.

The solid phase substance is preferable to a particle. By using a particle as a solid phase substance and adding the particle to a blood sample, the red blood cell can be stuck on the surface of the particle. The particle is dispersed in the blood sample, which is advantageous in that the particle can efficiently stick the red blood cells. Hereinafter, the particle as a solid phase substance is referred to as a solid phase particle.

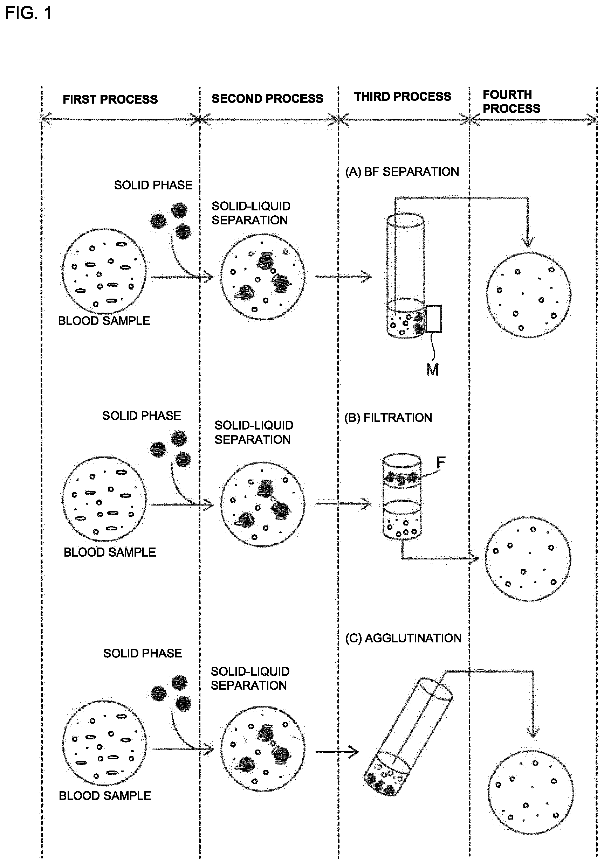

An embodiment of removing the red blood cell using a solid phase particle is described with reference to FIG. 1. An embodiment of a method of removing the red blood cell using the solid phase particle includes (A) BF (Bound Free) separation method, (B) filtration method and (C) agglutination method.

The BF separation method is described. In the BF separation method, a magnetic particle is used as a solid phase particle. As illustrated in FIG. 1, in a first process, a magnetic particle with the red blood cell capture antibody fixed on its surface is added to a blood sample. A mixture of the blood sample and the magnetic particles is preferably stirred to disperse the magnetic particles in the blood sample. In a second process, the red blood cell in the blood sample is stuck onto the surface of the magnetic particles. In a third process, the magnetic particles are attracted by a magnet. For example, as illustrated in FIG. 1, a magnet M is provided near a container that stores the mixture of the blood sample and the magnetic particles (a side in an embodiment of FIG. 1), and the magnetic particles dispersed in the container are localized on an inner surface of the container. This separates the red blood cell in the container. In a fourth process, a portion of liquid in the container with the magnetic particles localized, which does not include the red blood cell, for example, a supernatant, is separated. Therefore, the red blood cell is separated from the blood sample.

The time for providing the magnet M on the side of the container to attract the magnetic particles is preferably one minute or longer, more preferably five minutes or longer. A position at which the magnet M is provided may be near the container and at a position where the supernatant can be aspirated by pipetting avoiding a complex including the localized magnetic particles while providing sufficient magnetic force to localize the magnetic particles in the container, for example at the side or bottom of the container. The third process of providing the magnet M near the container can be achieved by moving the magnet M and the container relative to each other. For example, a container including magnetic particles can be moved closer to the magnet M, or the magnet M can be moved closer to the container.

The BF separation method has an advantage of being easy to operate because the magnetic particles can be attracted to the side of the container to separate the red blood cell in a liquid phase.

Next, the filtration method is described. In the filtration method, as in the first and second processes of the BF separation method, the solid phase particle is added to the blood sample, and the red blood cell is stuck on the surface of the solid phase particle. In a third process, the blood sample including the solid phase particle with the red blood cell stuck is dispensed into a column with a filter F. The filter F comprises a porous member having a pore size that allows a target cell, e.g. a white blood cell, to pass through but not the solid phase particle. When the blood sample is dispensed onto a top surface of the filter, the blood sample is filtered by the filter F, the target cell passes through the filter F, and the solid phase particle with the red blood cell stuck is trapped in the filter F. Therefore, the red blood cell is separated from the blood sample including the target cell.

Although the filtration method illustrates an embodiment using the filter F, a bead column may be used instead of a filter. In case that a bead column is used, a solid phase particle, in which the red blood cell capture antibody is fixed on its surface, is filled in the column, and the blood sample is injected into the column. When the blood sample passes through a gap between the solid phase particles, the red blood cell included in the blood sample is stuck on the surface of the solid phase particle.

Next, the agglutination method is described. In the agglutination method, as in the first and second processes of the BF separation method, the solid phase particle is added to the blood sample. The red blood cell is stuck on the surface of the solid phase particle, and the red blood cell and the solid phase particle form an agglomerate. In a third process, the blood sample including the solid phase particle with the red blood cell stuck is left to stand for a predetermined time. The agglomerate formed by the red blood cell and the solid phase particle settles to the bottom of the container by its own weight. In this state, the red blood cell is separated from the blood sample by separating the supernatant including the target cell.

In the agglutination method, it is preferable to select a solid phase particle that easily forms red blood cells and an agglomerate, and is preferable to use a latex particle, for example.

<Immunostaining>

As described above, cells in the blood sample from which the red blood cell is removed is immunostained. In immunostaining, for example, by mixing an antibody reagent that includes a labeled antibody capable of binding to a target antigen of a target cell with a blood sample, the target antigen is labeled.

The labeled antibody is, for example, an antibody modified by a labeled molecule that produces a fluorescent signal of a specific wavelength in response to being irradiated with an excitation light having a specific central wavelength.

The number of antigens to be labeled by immunostaining is not particularly limited, and may be one or more than one type. In case that multiple types of antigens are stained simultaneously, multiple target antigens can be stained by mixing a cocktail reagent that includes multiple labeled antibodies, which are labeled to emit different fluorescent signals from each other, with a blood sample.

The type of antigen may be an antigen expressed on a cell surface, an antigen expressed intracellularly, or a combination thereof. The type of antigen to be combined can be changed according to the target antigen. For example, in an embodiment described below, Treg cell and Teff cell are the target cells. Teff cells can be identified as a CD4 antigen positive T cell (CD4+T cell) with low expression of CD62L antigen. Also, Treg cell can be identified as a CD4+T cell that are CD25 antigen positive and FOXP3 antigen positive. In an embodiment, CD4, CD62L, and CD25 can be immunostained as cell surface antigens, and FOXP3 can be immunostained as an intracellular antigen.

As a result of the inventors' examination, among the antigen exemplified above, CD62L, CD25, and FOXP3 are found to have reduced fluorescent signals when treated with lysing agents to remove the red blood cell in a blood sample. This may be due to a stimulation or damage of cell surface and intracellular antigens by the lysing agent affecting the cell. In contrast, when a method of removing the red blood cell by using a solid phase substance that can bind to the red blood cell instead of a lysing agent is employed, it confirmed that the effect of the lysing agent on the antigen signal described above can be reduced.

When the cell surface antigen (e.g. CD4, CD62L, and CD25) and the intracellular antigen (e.g. FOXP3 ) are stained by immunostaining, it is preferable to include a process of mixing a first antibody reagent that includes a labeled antibody, which specifically binds to a cell surface antigen, and a second antibody reagent that includes a labeled antibody, which specifically binds to an intracellular antigen, with a blood sample. Further, the order of mixing with a blood sample is preferably in the order of the first antibody reagent and the second antibody reagent. It is preferable that the blood sample is stained for the cell surface antigen by the first antibody reagent and then mixed with a fixation agent and a membrane permeabilization agent before an addition of the second antibody reagent. Since a large molecule such as the labeled antibody cannot pass through a cell membrane and enter inside of a cell, the membrane permeabilization agent allows the labeled antibody to pass through the cell membrane and enter the cell. The fixation agent fixes the intracellular antigen, or protein, not to diffuse through the cell membrane treated by the membrane permeabilization agent.

<Measurement by flow cytometer>

An immunostained measurement specimen is subjected to a measurement by a flow cytometer. The flow cytometer obtains an optical signal, such as a fluorescent signal, from an immunostained cell by the flow cytometry method, and analyzes the cell based on the fluorescent signal.

The following is a description of a method of classification and counting of Treg cell and Teff cells when a measurement specimen, in which the red blood cell is removed by the above method and the cell is immunostained, is measured by the flow cytometer.

First, a lymphocyte is classified from the white blood cell in a blood sample. The lymphocyte is classified based on a parameter based on, for example, forward scattered light intensity and lateral scattered light intensity.

Next, among the lymphocytes, cells having a CD4+signal a predetermined value or over are classified. Specifically, cells in which the fluorescent signal emitted from the labeled antibody binding to the CD4 antigen is a predetermined value or over are identified. To enhance the classification performance, in addition to the fluorescent signal corresponding to CD4, for example, scattered light intensity, more preferably lateral scattered light intensity, may be combined as a parameter.

Among CD4+cells, cells with low expression of CD62L (CD62L$^{low}$) are classified. Specifically, a cell, in which the fluorescent signal emitted from the labeled antibody binding to the CD62L antigen is a predetermined value or less, is identified. When a histogram of the intensity of the fluorescent signal emitted from the labeled antibody binding to the CD62L antigen is plotted on a horizontal axis and the number of cells corresponding to the intensity is plotted on the vertical axis is made, the peak of cells with high CD62L expression appears on a high value side of the fluorescent signal, and the peak of cells with low CD62L expression appears on a low value side of the fluorescence signal; therefore, a so-called bimodal histogram is drawn. A cell that is plotted on the low value side of the fluorescent signal below a valley between these two peaks is identified as CD62L$^{low}$.

Among the CD4+cells, cells with FOXP3+ and CD25+ are classified. Specifically, a cell, in which the fluorescent signal emitted from the labeled antibody binding to the FOXP3 antigen (FOXP3 signal) is a predetermined value or over and the fluorescent signal emitted from the labeled antibody binding to the CD25 antigen (CD25 signal) is a predetermined value or over, is identified. When a two-dimensional distribution plot is created with the intensity of the FOXP3 signal as a first axis and the intensity of the CD25 signal as a second axis, four quadrants are obtained, divided into regions the predetermined value or over and below the predetermined value for each of the two signals. Among these, a cell plotted in a quadrant where the FOXP3 signal is the predetermined value or over and the CD25 signal is the predetermined value or over is identified as a cell with FOXP3+ and CD25+. Therefore, the number and percentage of Treg cell and Teff cell in the blood are calculated. The percentage of Treg cells and the percentage of Teff cells in the blood are found to be effective in predicting the efficacy of an immune checkpoint inhibitor (nivolumab).

Specifically, the percentage of CD4+ and CD62L$^{low}$ cells to lymphocytes is calculated as %Teff, and the percentage of CD4+ and FOXP3+ and CD25+ cells to lymphocytes is calculated as %Treg, which can be substituted into the following formula (1) to obtain an index for predicting drug efficacy.

$$\text{Index} = [\%\text{Teff}]^2 / [\%\text{Treg}] \ldots (1)$$

For example, by comparing the index with an empirically set cutoff, it is possible to stratify patients into a response group and a non-response group of nivolumab. According to the specimen preparation method according to the present invention, fluorescent signals from Treg cell and Teff cell can be accurately detected, and an accurate counting result can be obtained.

<Automation by a pretreatment apparatus>

The method of preparing a measurement specimen of the present embodiment has the advantage of reducing the influence of the fluorescent signal on the target cell and further has an advantage of being easy to automate. The following describes an embodiment of automation of the preparation of a measurement specimen when a magnetic particle is used as a solid phase substance that can bind to the red blood cell.

[Overview of the specimen preparation apparatus]

Referring to FIG. 2, an overview of a specimen preparation apparatus 100 according to one embodiment is described.

As illustrated in FIG. 2, the specimen preparation apparatus 100 includes a first processing section 20 and a second processing section 30. The first processing section 20 performs a process to remove red blood cells from a blood sample using a solid phase substance capable of binding to red blood cells. The second processing section 30 performs a process to react between the blood sample from which the red blood cell is removed and a reagent to immunostain cells.

The first processing section 20 includes a processing container transfer section (rack 10a) that holds a processing container 11, a reaction container supply section (rack 10b) that holds a reaction container 12, and a sample setting section (rack 10c) that holds a sample container 13 in which a blood sample is stored. The first processing section 20 includes a first moving section 15, a second moving section 16, and a third moving section 17 to move the racks 10a, 10b, 10c in a lateral direction (X1 and X2 directions), respectively.

The specimen preparation apparatus 100 includes a first dispenser 41 and a container transfer section 42. The first dispenser 41 and the container transfer section 42 are movably supported in a Y direction on a common transfer axis 43. The first dispenser 41 includes a pipette 41a. The first dispenser 41 dispenses a blood sample stored in the sample container 13 into the processing container 11 using the pipette 41a. The container transfer section 42 includes a pair of hands 42a that can be brought close to and separated from each other. The container transfer section 42 holds the reaction container 12 by the hands 42a and transfers the reaction container 12 between the rack 10b and the second processing section 30.

The specimen preparation apparatus 100 includes an agitator 50 to agitates a blood sample. The agitator 50 removes the sample container 13 from the rack 10c and inverts and agitates the sample container 13 to agitate the blood sample.

The specimen preparation apparatus 100 includes a second dispenser 60. The second dispenser 60 is movably supported by a transfer axis 61 in the lateral direction (X1 and X2 directions). The transfer axis 61 is movably supported in the Y direction by a transfer axis 62. Thereby, the second dispenser 60 is movable in a horizontal direction in the apparatus. The second dispenser 60 includes a pipette 60a. The second dispenser 60 aspirates a supernatant from the processing container 11 using the pipette 60a and dispenses the supernatant into the reaction container 12. Also, the second dispenser 60 dispenses a reagent to be set in a reagent setting parts 70a and 70b described below into the processing container 11 of the first processing section 20 or the reaction container 12 in the second processing section 30.

The specimen preparation apparatus 100 includes the reagent setting part 70a and the reagent setting part 70b. The reagent setting part 70a includes a cold storage and keeps a reagent cool. The reagent setting part 70b holds a reagent at room temperature.

The specimen preparation apparatus 100 a nozzle cleaner 80. The nozzle cleaner 80 cleans a nozzle of the second dispenser 60.

The specimen preparation apparatus 100 includes a controller 90 that controls each part of the apparatus. The controller 90 includes a processor and a memory. The processor comprises, for example, a CPU. The memory may include a memory and a storage. The processor functions as the controller 90 of the specimen preparation apparatus 100 by executing a program stored in the memory.

The first processing section 20 includes a magnet 21 at a position adjacent to the first moving section 15. More specifically, the magnet 21 is provided adjacent to a side of the processing container 11 held in the rack 10a when the rack 10a holding the processing container 11 is moved by the first moving section 15 to the origin (the most right side (X2 side)). In an embodiment of FIG. 2, the magnet 21 is a permanent magnet, but the magnet 21 may also be an electromagnet.

The second processing section 30 is a centrifugation section that can hold multiple reaction containers 12, which are centrifuge tubes, in a circumferential direction and rotates at high speed around its axis to perform centrifugation of a specimen in the held reaction containers 12. The second processing section 30 includes a rotor 31 that rotates at a high speed and a plurality of holders 32 that are provided around the periphery of the rotor 31. The holder 32 has, for example, a cylindrical shape and can receive the reaction container 12 therein. Also, the holder 32 holds the reaction container 12 in an attitude in which the opening thereof faces upward when the rotor 31 is stopped.

[Overview of the method of preparing a measurement specimen]

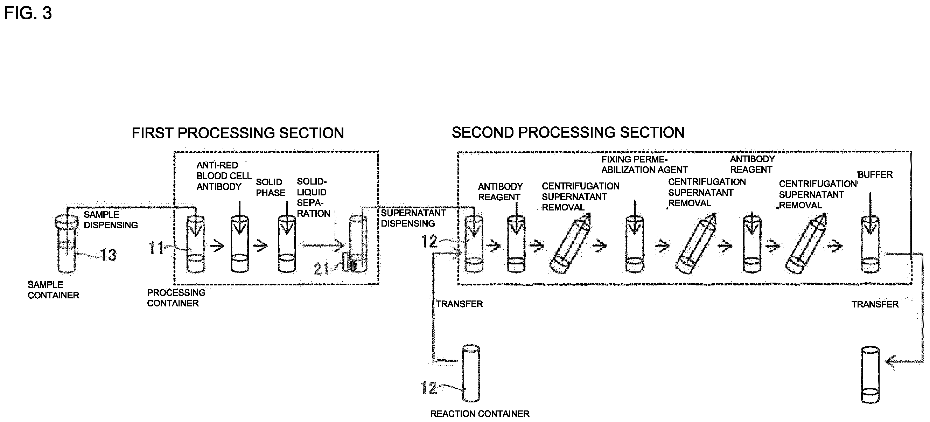

Referring to FIG. 3, an overview of the method of preparing a measurement specimen by the specimen preparation apparatus 100 is described.

The first dispenser 41 dispenses a portion of a blood sample that is stored in the sample container 13 held in the rack 10c into the processing container 11 held in the rack 10a. The second dispenser 60 dispenses a reagent that includes a biotinylated anti-red blood cell antibody into the processing container 11, into which the blood sample is dispensed. Also, the rack 10b transfers the reaction container 12 to the second processing section 30 by the container transfer section 42.

The second dispenser 60 dispenses a reagent that includes a streptavidin-bound magnetic particle as a solid phase substance into the processing container 11, into which the blood sample is dispensed. The streptavidin-bound magnetic particle react and bind with the biotinylated anti-red blood cell antibody; therefore, a complex including the red blood cell and the solid phase substance is formed in the processing container 11. The first moving section 15 positions the processing container 11 to the side of the magnet 21, so that the complex is collected at the side of the processing container 11.

The second dispenser 60 aspirates supernatant from the processing container 11, in which the complex is collected on the side of the processing container 11, and dispenses the supernatant into the reaction container 12 of the second processing section 30. Therefore, the supernatant including the cell to be measured is separated from the red blood cell included in the complex.

The second dispenser 60 dispenses an antibody reagent into the reaction container 12 into which the blood sample is dispensed. The antibody reagent is an antibody cocktail reagent that includes a labeled antibody, which binds to a cell surface antigen of a white blood cell (e.g., CD4, CD25, CD62L). After the reaction of the white blood cell in the specimen with the antibody reagent, the second processing section 30 performs centrifugation on the specimen in the reaction container 12. Centrifugation causes the white blood cell to settle to the bottom of the reaction container 12. The second dispenser 60 aspirates and removes the supernatant of the specimen after centrifugation. Therefore, the white blood cell that reacts with the labeled antibody remains in the reaction container 12, and the supernatant including an unreacted antibody component is removed.

The second dispenser 60 dispenses a fixation/permeabilization agent into the reaction container 12. The fixation/permeabilization agent fixes a protein in a cell membrane, and a membrane is permeabilized to allow entry of the labeled antibody into the cell membrane. After reaction with the fixation/permeabilization agent, the second processing section 30 centrifuges the specimen in the reaction container 12. Centrifugation causes the white blood cell to settle to the bottom of the reaction container 12. The second dispenser 60 aspirates and removes the supernatant of the specimen after centrifugation. Therefore, the white blood cell treated by the fixation/permeabilization agent remains in the reaction container 12, and the supernatant including the fixation/permeabilization agent is removed.

The second dispenser 60 dispenses the antibody reagent into the reaction container 12 by the second dispenser 60. The antibody reagent is a reagent that includes a labeled antibody that binds to an intracellular antigen (e.g., a labeled antibody that binds to FOXP3). After the reaction of the white blood cell in the specimen with the antibody reagent, the second processing section 30 performs centrifugation on the specimen in the reaction container 12. Centrifugation causes the white blood cell to settle to the bottom of the reaction container 12. The second dispenser 60 aspirates and removes the supernatant of the specimen after centrifugation. Therefore, the white blood cell that reacts with the labeled antibody remains in the reaction container 12, and the supernatant including an unreacted antibody component is removed.

The second dispenser 60 dispenses a buffer (e.g., phosphate buffer) into the reaction container 12. Therefore, a measurement specimen is prepared. The container transfer section 42 transfers the reaction container 12, in which the measurement specimen is stored, to the rack 10b.

[Operation of the specimen preparation apparatus]

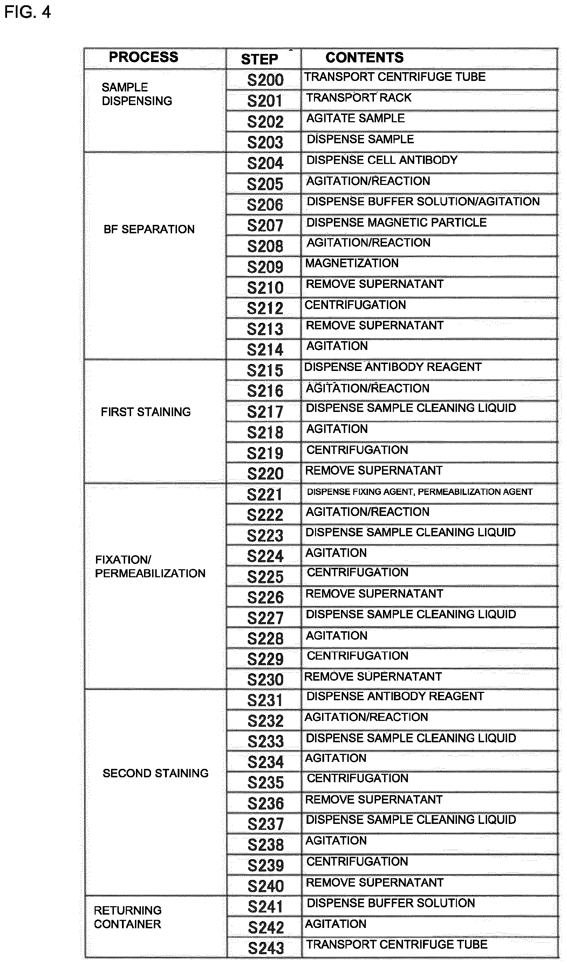

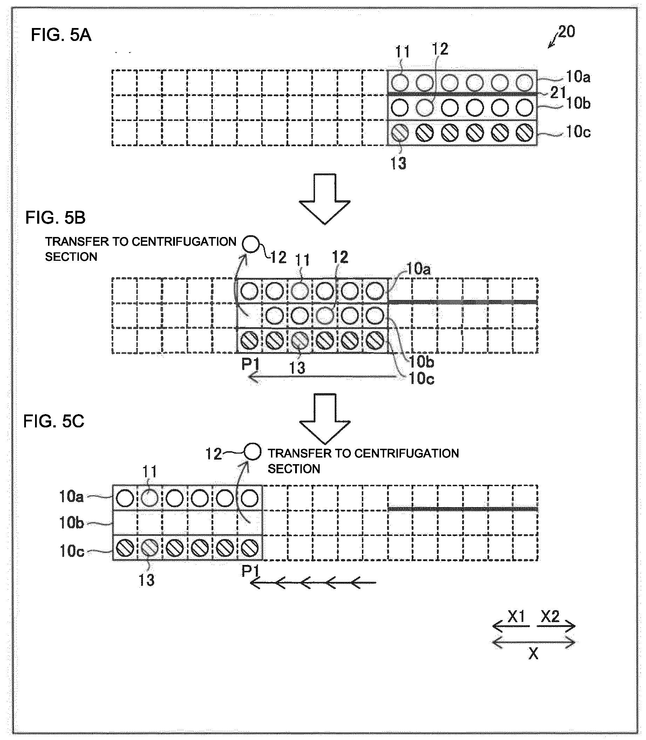

Referring to FIG. 4, an operation of a measurement specimen by the specimen preparation apparatus 100 is described. In the following, movements of the racks 10a to 10c by the first moving section 15, the second moving section 16, and the third moving section 17 are also referred to in FIG. 5 to FIG. 8.

As illustrated in FIG. 5, the racks 10a, 10b, and 10c can each hold a plurality of containers (e.g., six). The racks 10a, 10b, and 10c are transported in an X1 direction and an X2 direction by the first moving section 15, the second moving section 16, and the third moving section 17. This embodiment describes an embodiment, in which the first moving section 15, the second moving section 16, and the third moving section 17 integrally move the racks in the X1 direction and the X2 direction; however, the embodiment may be an embodiment, in which the first moving section 15, the second moving section 16, and the third moving section 17 may independently move the racks in the X1 direction and the X2 direction. The first moving section 15, the second moving section 16, and the third moving section 17 can transport the racks 10a, 10b, and 10c in the X1 direction and the X2 direction by a distance corresponding to the spacing of the containers held in the racks. In FIG. 5 to FIG. 8, in order to illustrate positions of each container in a clear manner, squares of a size corresponding to the spacing of the containers are illustrated.

[Sample dispensing process]

Prior to a sample dispensing process, as illustrated in FIG. 5A, an empty processing container 11 is set in the rack 10a by an operator. Also, an empty reaction container 12 is set in the rack 10b by the operator. A sample container 13, in which a blood sample is stored, is set in the rack 10c by the operator.

In step S201 of FIG. 4, a centrifuge tube as the reaction container 12 is transported from the rack 10b to the second processing section 30. As illustrated in FIG. 5B, the rack is moved in the X1 direction so that the leftmost reaction container 12 is located at a position P1.

The position P1 is the twelfth square from the rightmost position. At the position P1, the reaction container 12 is removed by the container transfer section 42 and transferred to the second processing section 30. As illustrated in FIG. 5C, the rack is moved in the X 1 direction so that the next reaction container 12 is located at the position P1. Then, the reaction containers 12 are taken out by the container transfer section 42 and sequentially transferred to the second processing section 30. This process is repeated until the last reaction container 12 is moved to the position P1 and transferred to the second processing section 30 by the container transfer section 42.

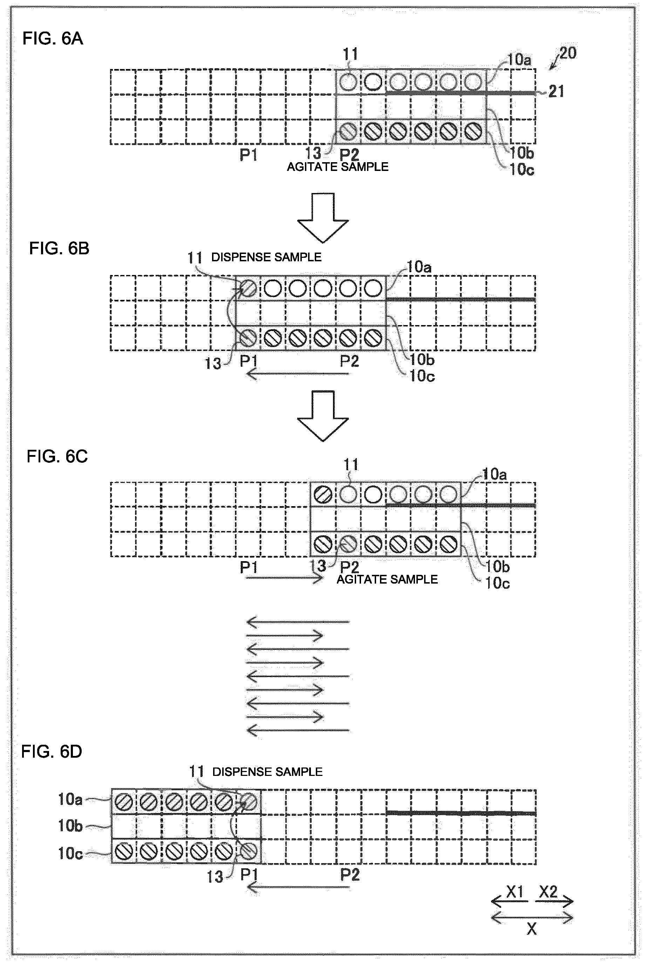

In step S202, a blood sample in the sample container 13 is agitated. As illustrated in FIG. 6A, the rack is moved in the X2 direction by the third moving section 17 so that the most left sample container 13 is located at a position P2. The position P2 is the eighth square from the rightmost position. At the position P2, the sample container 13 of the rack 10c is removed by the agitator 50 and inverted and agitated.

In step S203, a blood sample is aspirated from the sample container 13 and discharged into the processing container 11 of the rack 10a to dispense the blood sample. As illustrated in FIG. 6B, the rack is moved in the X1 direction so that the sample container 13 agitated in step S202 is located at the position P1. At the position P1, the first dispenser 41 aspirates a portion of the blood sample in the sample container 13 and dispenses the aspirated blood sample into the processing container 11 held in the rack 10a. As illustrated in FIG. 6C, the rack is moved in the X2 direction so that the next sample container 13 is located at the position P2. At the position P2, the sample container 13 is agitated by the agitator 50. This agitation of the sample container 13 at the position P2 and subsequent dispensing of the blood sample at the position P1 are repeated until the agitation and the dispensing are performed on all sample containers 13. FIG. 6D is a diagram illustrating a state in which the agitation and the dispensing on all sample containers 13 are completed.

<BF separation process>

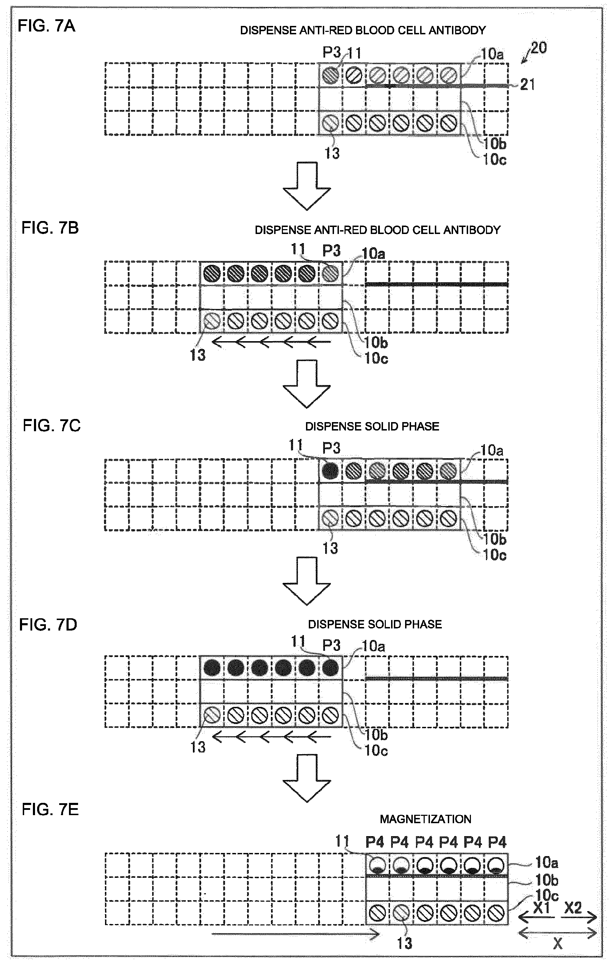

In step S204, the antibody is dispensed into the processing container 11 from which the blood sample is dispensed. As illustrated in FIG. 7A, the rack is moved in the X2 direction so that the leftmost processing container 11 is located at a position P3. At the position P3, the second dispenser 60 dispenses the biotinylated anti-red blood cell antibody into the processing container 11. As illustrated in FIG. 7B, the rack is moved in the X1 direction so that the next processing container 11 is located at the position P3. Then, at the position P3, the process of dispensing the anti-red blood cell antibody into the processing container 11 by the second dispenser 60 is repeated.

When dispensing into all the processing containers 11 is completed, in step S205, an agitation of the blood sample in the processing container 11 is performed. The rack is repeatedly moved back and forth in the X1 direction and the X2 direction by the first moving section 15, and the blood sample in the processing container 11 is agitated. The rack 10a holding the processing container 11 is then allowed to stand for a predetermined time (e.g. 20 minutes).

In step S206, a buffer solution is dispensed into the processing container 11. The first moving section 15 moves the rack 10a, and at the position P3, a buffer is dispensed into each processing container 11 by the second dispenser 60. The rack 10a is moved at high speed by the first moving section 15, and the blood sample in the processing container 11 is agitated. Then, the blood sample is allowed to stand still. For example, BSA solution and phosphate buffered saline (PBS) are dispensed as buffer solutions. Also, the blood sample to which the buffer solution is dispensed is agitated.

In step S207, a magnetic particle is dispensed into the processing container 11. For example, a streptavidin-bound magnetic particle is dispensed as a magnetic particle. As illustrated in FIG. 7C, the rack is moved in the X1 direction so that the leftmost processing container 11 is located at a position P3. At the position P3, the streptavidin-bound magnetic particle as the solid phase substance is dispensed into the processing container 11 by the second dispenser 60. As illustrated in FIG. 7D, the rack is moved in the X1 direction so that the next processing container 11 is located at the position P3. Then, at the position P3, a process of dispensing the solid phase substance including the magnetic particle into the processing container 11 by the second dispenser 60 is repeated.

In step S208, the blood sample in the processing container 11 is agitated, and a reaction is performed. The time required is, for example, 5 minutes. The rack is moved at high speed by the moving section 15, and the blood sample in the processing container 11 is agitated. Then, the blood sample is allowed to stand still. Therefore, a complex including the red blood cell and the solid phase substance is formed in the processing container 11.

In step S209, magnetic attraction is performed. As illustrated in FIG. 7E, the rack is moved in the X 2 direction so that each processing container 11 is located at a position P4. The position P4 is a position corresponding to six squares from the rightmost square. A magnet 21 is provided at a position adjacent to the position P4. The magnet 21 causes the complex including the red blood cell and the solid phase substance to be attracted on the inner surface of the processing container 11. The time required is 10 minutes, for example.

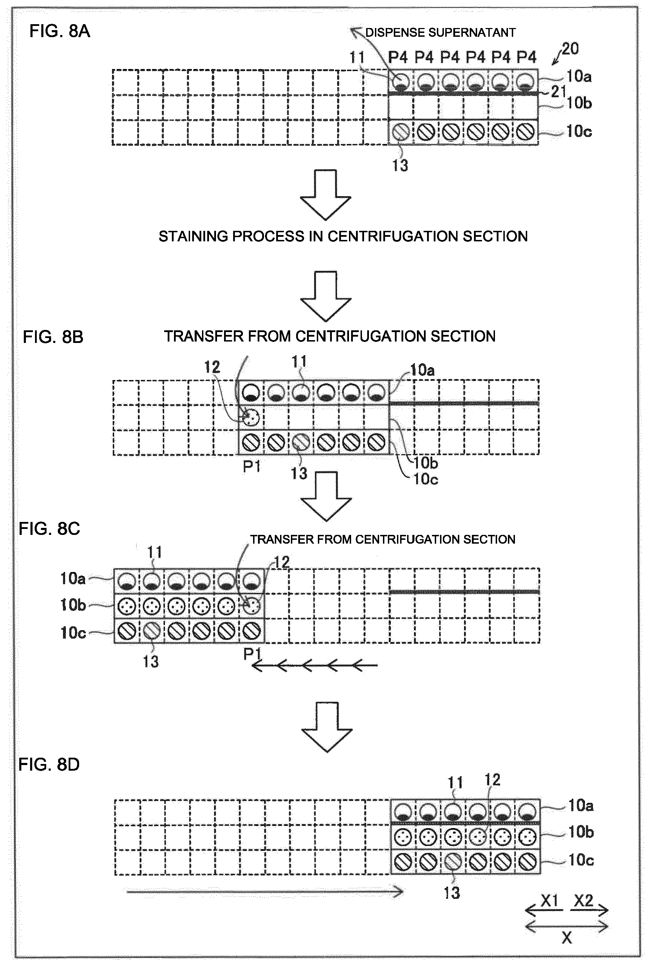

In step S210, the supernatant (e.g., 700 μL) of the blood sample in the processing container 11 where magnetic attraction takes place is aspirated and discharged and dispensed into the reaction container 12 in the second processing section 30. As illustrated in FIG. 8A, at the position P4, the supernatant is aspirated from each processing container 11 by the second dispenser 60 and dispensed into each reaction container 12 that is transferred to the second processing section 30.

In step S212, the specimen in the reaction container 12 is centrifuged. In the second processing section 30, the rotor 31 is rotated at a high speed to settle the white blood cell at the bottom of the reaction container 12.

In step S213, the supernatant in the reaction container 12 is removed. The second dispenser 60 aspirates and removes the supernatant (e.g., 600 μL) of the reaction container 12, in which the white blood cell is settled by centrifugation.

In step S214, the specimen in the reaction container 12 is agitated. The steps S212 and S213 allow the white blood cell to settle at the bottom of the reaction container 12. In order to disperse the settled white blood cell, the reaction container 12 is agitated. The second processing section 30 agitates the specimen in the reaction container 12 by rotating the rotor 31 while repeatedly accelerating and decelerating in one direction.

<First staining process>

In step S215, the antibody reagent is dispensed into the reaction container 12. The second dispenser 60 dispenses the cocktail reagent including CD25-labeled antibody, CD4-labeled antibody, and CD62L-labeled antibody as the antibody reagent into the reaction container 12 held in the second processing section 30.

In step S216, the second processing section 30 agitates the specimen in the reaction container 12 by rotating the rotor 31 in one direction with repeated acceleration and deceleration. The predetermined time is, for example, 30 minutes.

In step S217, the reaction container 12 is dispensed with a cleaning liquid for the sample before fixation. The second dispenser 60 dispenses the PBS as the cleaning liquid into the reaction container 12.

In step S218, the second processing section 30 agitates the specimen in the reaction container 12 by rotating the rotor 31 in one direction with repeated acceleration and deceleration.

In step S219, the second processing section 30 centrifuges the specimen in the reaction container 12 by rotating the rotor 31 at a high speed in one direction. As a result, the white blood cell that reacts with the antibody reagent is settled.

In step S220, the second dispenser 60 aspirates and removes the supernatant from the reaction container 12. As a result, the surface antigens CD25, CD4 and CD62L are stained with the corresponding labeled substances.

<Processes of fixation and permeabilization of cells>

In step S221, the second dispenser 60 dispenses a fixation/permeabilization agent into the reaction container 12.

In step S222, the second processing section 30 agitates the specimen in the reaction container 12 by rotating the rotor 31 in one direction with repeated acceleration and deceleration, and the reaction is performed. The predetermined time is, for example, 30 minutes.

In step S223, the second dispenser 60 dispenses a cleaning liquid for the post-fixation sample into the reaction container 12.

In step S224, the second processing section 30 agitates the specimen in the reaction container 12 by rotating the rotor 31 in one direction with repeated acceleration and deceleration.

In step S224, the second processing section 30 centrifuges the specimen in the reaction container 12 by rotating the rotor 31 at a high speed.

In step S226, the second dispenser 60 aspirates and removes the supernatant from the reaction container 12.

In steps S227 to S230, dispensing of the cleaning liquid, agitation, centrifugation and removal of the supernatant are performed. In other words, a cleaning process of the sample is repeated. The cleaning process of the sample may be performed once, twice, three or more times. In the above, the fixation process and the permeabilization process are performed for the cell in the reaction container 12.

<Second staining process>

In step S231, the second dispenser 60 dispenses the antibody reagent into the reaction container 12. For example, a reagent including an anti-FOXP3-labeled antibody is dispensed as the antibody reagent.

In step S232, the second processing section 30 rotates the rotor 31 in one direction with repeated acceleration and deceleration, thereby agitating the specimen in the reaction container 12 and causing a reaction. The predetermined time is, for example, 30 minutes.

In step S233, the second dispenser 60 dispenses a cleaning liquid for the post-fixation sample into the reaction container 12. In steps S234 to S236, agitation, centrifugation, and removal of the supernatant are performed in the same manner as described above, and in steps S237 to S240, a process of cleaning for the sample, which includes dispensing of the cleaning liquid, agitation, centrifugation and removal of the supernatant, is repeated. The cleaning process of the sample may be performed once, twice, three or more times. Therefore, the FOXP3 in the reaction container 12 is stained with the corresponding labeled substance.

<Returning the container>

In step S241, the second dispenser 60 dispenses a buffer solution into the reaction container 12. By dispensing, the specimen in the reaction container 12 is adjusted to a predetermined amount of liquid and a predetermined pH suitable for supply to a measurement apparatus. For example, BSA solution and PBS are dispensed as buffer solutions.

In step S242, the second processing section 30 rotates a rotor in the same manner as described above to perform agitation.

In step S243, the container transfer section 42 removes the reaction container 12 held in the second processing section 30 from the rotor 31 and sets the reaction container 12 on the rack 10b held in the second transfer section 16. When all the reaction containers 12 are transferred to the rack 10b, as illustrated in FIG. 8D, the moving sections 15 to 17 move the rack in the X2 direction to return to an initial position. This enables an operator to remove the reaction container 12.

As a result of the above, a specimen preparation by the specimen preparation apparatus 100 is completed.

<Comparative data of a separation method and a hemolysis method>

The following experiment shows an effect of a separation method according to the present invention and a hemolysis method according to a comparative example on the detection performance of Treg cell and Teff cell.

(Example (specimen preparation by the separation method))

A whole blood sample collected in a heparin blood collection tube is dispensed into a test tube. 50 $\mu$L of biotinylated CD235a antibody is added to the test tube, and the test tube is agitated and allowed to stand for 20 minutes at room temperature. 0.6 mL of phosphate buffered saline (PBS) is added to the test tube. 200 $\mu$L of a solution including the streptavidin-bound magnetic particle (Mojosort (registered trademark)) is added to the test tube, and the test tube is agitated and allowed to stand for 5 minutes at room temperature. A permanent magnet is provided in contact with a side of the test tube and allowed to stand for 10 minutes at room temperature to allow a complex of the red blood cell-biotinylated CD235a antibody-streptavidin-bound magnetic particle are collected on the inner wall of the test tube. 0.7 mL of the supernatant of the solution in the test tube is collected with a pipette and dispensed into a centrifuge tube. A specimen of an example is obtained by centrifuging the centrifuge tube at 300 G for 5 minutes at room temperature and removing 600 $\mu$L of the supernatant.

(Staining of a cell surface antigen)

50 $\mu$L of CD4/CD25/CD62L antibody cocktail reagent is added to the specimen, and the specimen is agitated and allowed to stand for 30 minutes at room temperature. The antibody cocktail reagent includes an FITC-labeled CD4 antibody and a PE-Cy7-labeled CD25 antibody, and an APC-labeled CD62L antibody. 1 mL of PBS is added and agitated to the specimen already reacted with the antibody cocktail reagent. The specimen is centrifuged at 300 G for 5 minutes at room temperature, and the supernatant is removed to agitate the specimen.

(Fixation/membrane permeabilization)

0.5 mL of fixation/permeabilization agent (eBioscience Foxp3 / Transcription Factor Fixation/Permeabilization Concentrate and Diluent, manufactured by Invitrogen) is added to the centrifuged specimen, and the specimen is agitated and allowed to stand for 30 minutes at room temperature. 1 mL of a cleaning liquid (eBioscience Permeabilization Buffer (10X), manufactured by Invitrogen) is added to the specimen, and the specimen is agitated. The specimen is centrifuged at 400 G for 5 minutes at room temperature, and the supernatant is removed. 1 mL of the cleaning liquid (same as above) is added to the centrifuged specimen to agitate the specimen, and the specimen is centrifuged at 400 G for 5 minutes at room temperature and agitated after removing the supernatant.

(Staining of an intracellular antigen)

50 μL of an antibody reagent including a PE-labeled FOXP3 antibody is added to an obtained specimen, and the specimen is allowed to stand for 30 minutes at room temperature. 1 mL of a cleaning liquid (eBioscience Permeabilization Buffer (10X), manufactured by Invitrogen) is added to the specimen, and the specimen is stirred. The specimen is centrifuged at 400 G for 5 minutes at room temperature, and the supernatant is removed. 1 mL of the cleaning liquid (same as above) is added to the centrifuged specimen to agitate the specimen, and the specimen is centrifuged at 400 G for 5 minutes at room temperature, and the supernatant is removed to agitate the specimen.

(Measurement by the flow cytometer)

0.3 mL of PBS including 0.5% BSA is added to the specimen to agitate the specimen. The obtained specimen is measured by a commercially available flow cytometer FACS Canto II (manufactured by Becton Dickinson), and Teff cell (CD62 $L^{low}$ and CD4+ T cell) and Treg cell (CD25+, Foxp3+ and CD4+ T cell) are counted respectively.

(Comparative example (specimen preparation by the hemolysis method))

A whole blood sample collected in a heparin blood collection tube is dispensed into a centrifuge tube. 2 mL of hemolytic agent (CyLyse; manufactured by Sysmex Corporation) is added to the centrifuge tube and allowed to stand for 10 minutes at room temperature to hemolyze the red blood cell in the blood. The centrifuge tube is agitated with a vortex mixer, and then 2 mL of PBS is added to the centrifuge tube. The centrifuge tube is centrifuged at 300 G for 5 minutes at room temperature, and the supernatant is removed. 2 mL of PBS is added to the centrifuge tube, the centrifuge tube is centrifuged again at 300 G for 5 minutes at room temperature, and the supernatant is removed to obtain a specimen of a comparative example. The specimen of the comparative example is subjected to the same operation as in an embodiment and measured by the flow cytometer.

(Control experiment)

5 ml of the whole blood sample collected in a heparin blood collection tube is diluted in 5 mL of a liquid medium (10 mM HEPES/PRMI-1640). 10mL of a lymphocyte isolation medium (Ficoll-Paque Plus, manufactured by Cytiva) is added to a 50m L tube. The diluted whole blood sample is put in this tube and layered. The PBMC is separated in the tube according to the instructions provided in the Ficoll Paque Plus attachment, and the PBMC is suspended to $1 \times 10^6$ cells/mL using CELLBANKER2 (manufactured by Nippon Zenyaku Kogyo Co., Ltd.) and frozen in a deep freezer. After the freezing process, the PBMC is transferred to liquid nitrogen (under liquid phase) within 24 hours or later within 1 week. A thawed cell is cleaned with 10% FBS/RPMI1640, and then 10% FBS/RPMI1640 is added to reach $1 \times 10^6$ cells/mL. 2mL of a cell suspension liquid is added to a 24-hole plate (manufactured by Corning Inc.) and incubated for 48 hours. After incubation, the cell culture medium is transferred to a 15-mL tube, and the tube is centrifuged at 400 G for 5 minutes to obtain a control specimen. The specimen of the comparative example is subjected to the same operation as in an embodiment and measured by the flow cytometer.

(Measurement result)

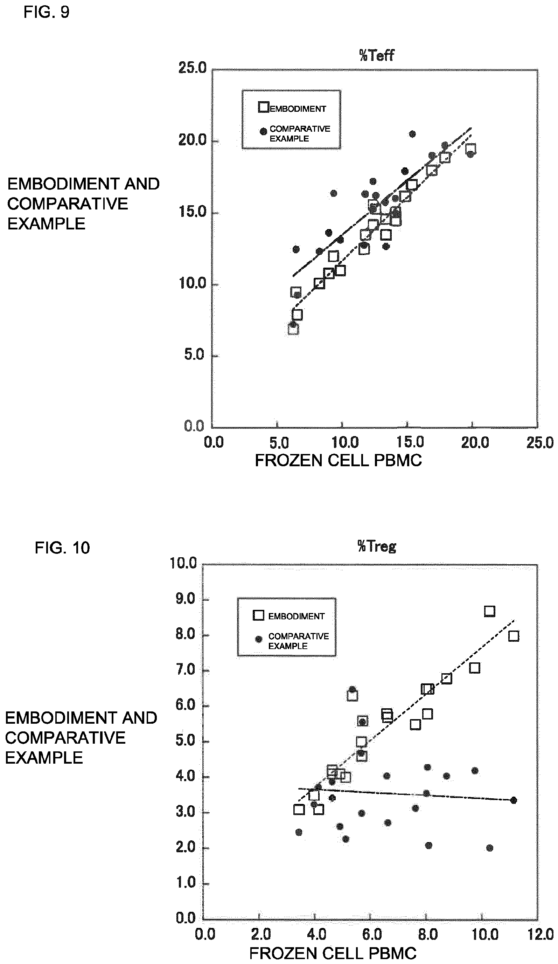

Measurement results are illustrated in FIG. 9 and FIG. 10.
As illustrated in FIG. 9, in terms of the detection performance of Teff cells, specimens for measurement prepared by the separation method of the example illustrates good correlation with specimens made from frozen PBMCs in a control experiment (correlation coefficient = 0.9419). Furthermore, the example of the embodiment illustrates a predominantly higher value in the correlation coefficient than that of the comparative example (correlation coefficient = 0.7064).
As illustrated in FIG. 10, in terms of the detection performance of Treg cells, specimens for measurement prepared by the separation method of the example illustrates good correlation with the results obtained with frozen PBMCs in the control experiment (correlation coefficient = 0.8679). Furthermore, the example of the embodiment illustrates a predominantly higher value in the correlation coefficient than that of the comparative example (correlation coefficient = 0.0065).

These results indicate that the separation method according to the present invention has higher measurement accuracy than the hemolysis method for both Teff cell and Treg cell. In addition, the separation method is found to have the same measurement accuracy as that of specimen preparation using the frozen PBMC.

(Consideration of a signal from labeling)

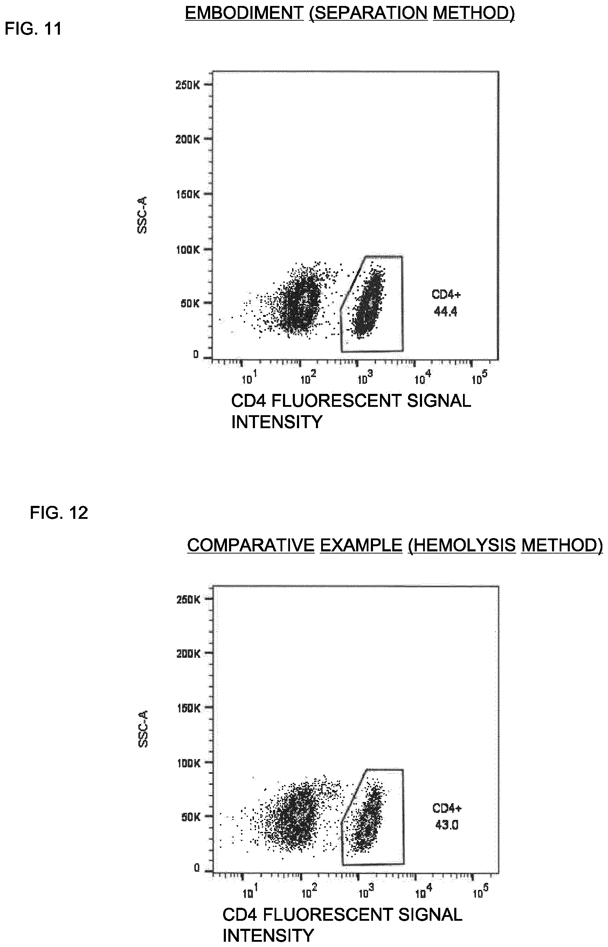

FIG. 11 and FIG. 12 are scattergrams illustrating fractionated T cells with a high CD4 expression level (CD4+T cell) among cells fractionated into lymphocytes. FIG. 11 is a scattergram illustrating the example (separation method), and FIG. 12 is a scattergram illustrating the comparative example (hemolysis method). The results of CD4+T cell counts are 44.4% for the example (separation method) and 43.0% for the comparative example (hemolysis method); therefore, there is no significant difference.

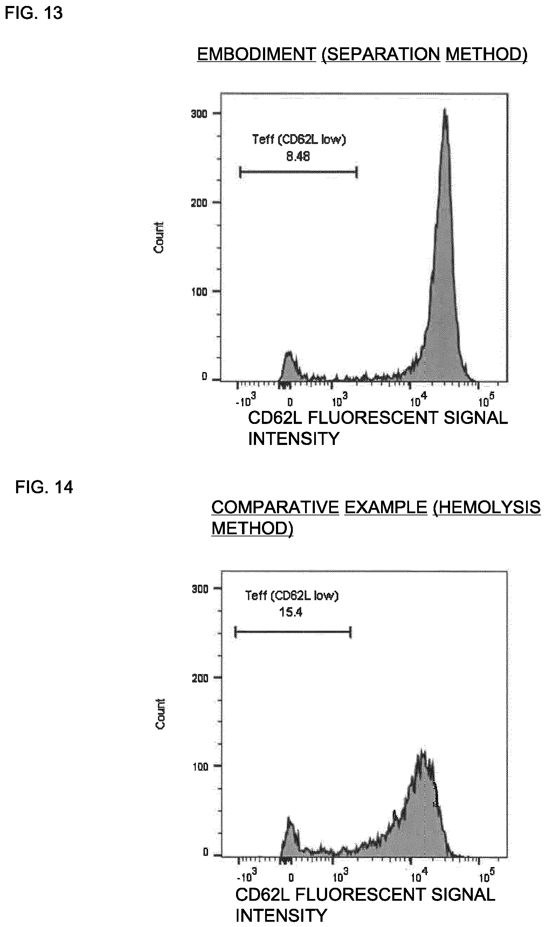

FIG. 13 and FIG. 14 are histograms obtained from the same sample, in which CD4+T cells with low CD62L expression levels (T cells of CD4+, CD62L$^{Low}$) are fractionated. FIG. 13 illustrates a histogram of the example (separation method), and FIG. 14 illustrates a histogram of the comparative example (hemolysis method). In this histogram, CD4+, CD62L$^{Low}$ T cells, or Teff cells, are counted when the CD62L fluorescent signal falls within the predetermined cutoff range. Teff cells are 8.48% in the example (separation method), whereas Teff cells are 15.4% in the comparative example (hemolysis method). In the histogram of the example (separation method), the peak of CD62L$^{Low}$ T cells and the peak of T cells with high CD62L expression levels (CD62L$^{High}$ T cells) are clearly separated, and few cells are plotted in a valley between the peaks. On the other hand, the histogram of the comparative example (hemolysis method) has a lower peak of CD62L$^{High}$ T cells and a higher number of cells plotted in the valley between the two peaks than the histogram of the example (separation method). This may be due to insufficient staining of CD62L in the hemolysis method, resulting in an overall decrease in fluorescence signal intensity and cells with high CD62L expression levels (CD62L$^{High}$ T cells) slide to the low side. The data suggests that the overall CD62L signal decreases in the hemolysis method.

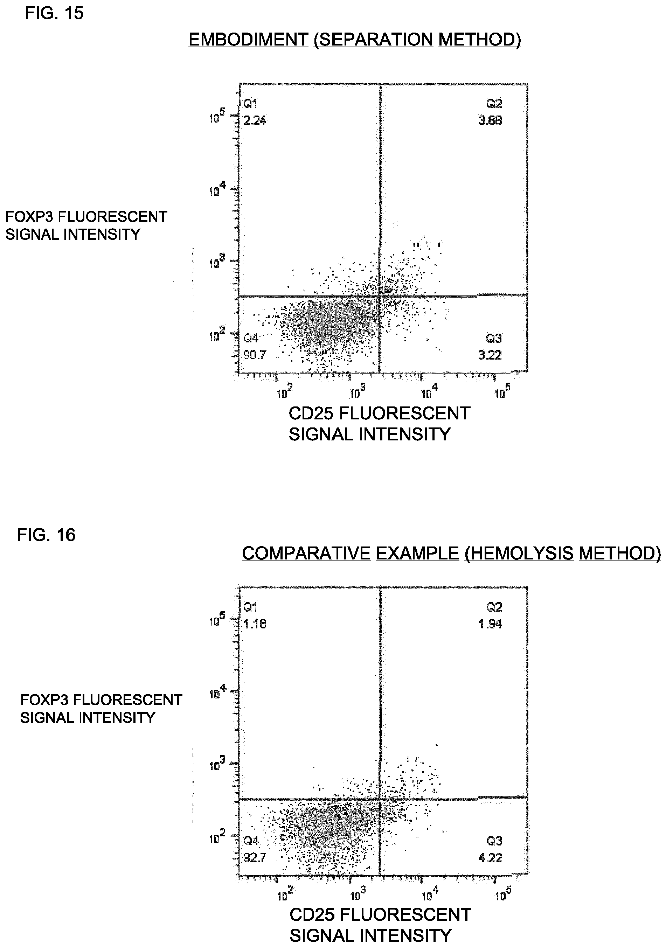

FIG. 15 and FIG. 16 are scattergrams from the same sample, in which CD4+T cells with high CD25 and FOXP3 expression levels (CD4+/CD25+/FOXP3+ T cells) are fractionated. FIG. 15 is a scattergram of the example (separation method), and FIG. 16 is a scattergram of the comparative example (hemolysis method). Cells that exhibited values higher than a predetermined cutoff values for the fluorescent signal intensity corresponding to CD25 (horizontal axis) and the fluorescent signal intensity corresponding to FOXP3 (vertical axis), respectively, are identified as CD25+/FOXP3+ T cells, or Treg cells (cells in a Q2 zone). Treg cells fractionated to Q2 are 3.88% in the example (separation method), whereas Treg cells fractionated to Q2 are 1.94% in the comparative example (hemolysis method). In the scattergram of the comparative example (hemolysis method), the number of cells in Q3 increases compared to the scattergram of the example (separation method). In other words, in the hemolysis method, the plots of cells are lowered in the vertical axis direction, suggesting that the signal of FOXP3 is decreased overall.

(Variation)

The embodiments disclosed herein should be considered exemplary and not restrictive in all respects. The scope of the invention is indicated by the claims rather than by the description of the above-described embodiments, and further includes all modifications within the meaning and scope equivalent to the claims.

[Description of the sign]

10a: Processing container transfer section, 10b: Reaction container supply section, 10c: Sample setting section, 11: Processing container, 12: Reaction container, 13: Sample container, 20: First processing section, 30: Second processing section, 41: First dispenser, 42: Container transfer section, 60: Second dispenser, 100: Specimen preparation apparatus

**Claims**

1. A method of preparing a measurement specimen comprising:

   removing red blood cells from a blood sample using a solid phase substance capable of binding to the red blood cells; and
   reacting the blood sample from which red blood cells are removed with a reagent to immunostain cells.

2. The method of preparing a measurement specimen according to claim 1, wherein
   the removing the red blood cell from the blood sample comprises:

   forming a complex comprising red blood cells and the solid phase substance; and
   separating the formed complex from a supernatant of the blood sample.

3. The method of preparing a measurement specimen according to claim 2, wherein
   the complex comprises red blood cells and the solid phase substance bound via a binding substance that comprises a capacity to bind to the red blood cell and the solid phase substance.

4.  The method of preparing a measurement specimen according to claim 3, wherein
the forming a complex comprising red blood cells and the solid phase substance comprises forming the complex by contacting the blood sample, the binding substance, and the solid phase substance.

5.  The method of preparing a measurement specimen according to claim 3 or 4, wherein
the binding substance comprises an antibody capable of binding to the red blood cell.

6.  The method of preparing a measurement specimen according to any one of claims 2 to 5, wherein

    the solid phase substance comprises a magnetic particle, and
    the removing red blood cells from the blood sample comprises aspirating the supernatant of the blood sample while attracting the complex with a magnet.

7.  The method of preparing a measurement specimen according to any one of claims 2 to 5, wherein
the removing red blood cells from the blood sample comprises separating the supernatant of the blood sample by filtering out the formed complex with a filter.

8.  The method of preparing a measurement specimen according to any one of claims 2 to 5, wherein
the removing red blood cells from the blood sample comprises separating the supernatant of the blood sample by settling the formed complex by centrifugation.

9.  The method of preparing a measurement specimen according to any one of claims 1 to 8, wherein
the reacting the blood sample from which red blood cells are removed with a reagent to immunostain cells comprises performing centrifugation of the blood sample.

10. A specimen preparation apparatus that prepares a measurement specimen by reacting a blood sample with a reagent comprising:

    a first processing section for removing red blood cells from a blood sample using a solid phase substance capable of binding to red blood cells; and
    a second processing section for reacting the blood sample from which red blood cells are removed with a reagent to immunostain cells.

11. The specimen preparation apparatus according to claim 10, wherein
the first processing section comprises a separating unit for forming a complex comprising red blood cells and the solid phase substance and separating the formed complex from a supernatant of the blood sample.

12. The specimen preparation apparatus according to claim 11, wherein
the complex comprises red blood cells and the solid phase substance bound together via a binding substance comprising a capacity to bind to the red blood cell and the solid phase substance.

13. The specimen preparation apparatus according to claim 12, wherein
the first processing section forms the complex by contacting the blood sample, the binding substance, and the solid phase substance.

14. The specimen preparation apparatus according to claim 12 or 13, wherein
the binding substance comprises an antibody capable of binding to the red blood cells.

15. The specimen preparation apparatus according to any one of claims 11 to 14, wherein

    the solid phase substance comprises a magnetic particle, and
    the first processing section comprises a magnet for attracting the complex and aspirates the supernatant of the blood sample while the complex is attracted by the magnet to separate the complex from the supernatant of the blood sample.

16. The specimen preparation apparatus according to any one of claims 11 to 14, wherein
the first processing section comprises a filter for filtering off the formed complex and separating the supernatant of the blood sample.

17. The specimen preparation apparatus according to any one of claims 11 to 14, wherein
the first processing section comprises a second centrifugation unit for separating the supernatant of the blood sample by settling the formed complex by centrifugation.

18. The specimen preparation apparatus according to any one of claims 10 to 17, wherein
the second processing section includes a first centrifugation unit for centrifuging the blood sample.

19. The specimen preparation apparatus according to any one of claims 10 to 18, further comprising:

    a sample setting section that a sample container storing the blood sample is placed;

a reaction container supply section that a reaction container storing the blood sample to be reacted in the second processing section is placed;

a container transfer section for transferring the reaction container between the reaction container supply section and the second processing section;

a first dispenser for dispensing the blood sample from the sample container into a processing container storing the blood sample to be processed in the first processing section; and

a second dispenser for dispensing a reagent installed in a reagent installation section into the processing container in the first processing section or into the reaction container in the second processing section.

20. The specimen preparation apparatus according to claim 19, wherein

the second dispenser aspirates a supernatant from the processing container and dispenses the supernatant into the reaction container in the second processing section.

FIG. 1

FIG. 2

FIG. 3

FIRST PROCESSING SECTION

SECOND PROCESSING SECTION

SAMPLE
DISPENSING

SAMPLE
CONTAINER

PROCESSING
CONTAINER

ANTI-RED
BLOOD CELL
ANTIBODY

SOLID
PHASE

SOLID-
LIQUID
SEPA-
RATION

SUPERNATANT
DISPENSING

ANTIBODY
REAGENT

CENTRIFUGATION
SUPERNATANT
REMOVAL

FIXING PERME-
ABILIZATION AGENT

CENTRIFUGATION
SUPERNATANT
REMOVAL

ANTIBODY
REAGENT

CENTRIFUGATION
SUPERNATANT
REMOVAL

BUFFER

TRANSFER

REACTION CONTAINER

TRANSFER

EP 4 235 143 A1

FIG. 4

| PROCESS | STEP | CONTENTS |
|---------|------|----------|
| SAMPLE DISPENSING | S200 | TRANSPORT CENTRIFUGE TUBE |
| | S201 | TRANSPORT RACK |
| | S202 | AGITATE SAMPLE |
| | S203 | DISPENSE SAMPLE |
| BF SEPARATION | S204 | DISPENSE CELL ANTIBODY |
| | S205 | AGITATION/REACTION |
| | S206 | DISPENSE BUFFER SOLUTION/AGITATION |
| | S207 | DISPENSE MAGNETIC PARTICLE |
| | S208 | AGITATION/REACTION |
| | S209 | MAGNETIZATION |
| | S210 | REMOVE SUPERNATANT |
| | S212 | CENTRIFUGATION |
| | S213 | REMOVE SUPERNATANT |
| | S214 | AGITATION |
| FIRST STAINING | S215 | DISPENSE ANTIBODY REAGENT |
| | S216 | AGITATION/REACTION |
| | S217 | DISPENSE SAMPLE CLEANING LIQUID |
| | S218 | AGITATION |
| | S219 | CENTRIFUGATION |
| | S220 | REMOVE SUPERNATANT |
| FIXATION/ PERMEABILIZATION | S221 | DISPENSE FIXING AGENT, PERMEABILIZATION AGENT |
| | S222 | AGITATION/REACTION |
| | S223 | DISPENSE SAMPLE CLEANING LIQUID |
| | S224 | AGITATION |
| | S225 | CENTRIFUGATION |
| | S226 | REMOVE SUPERNATANT |
| | S227 | DISPENSE SAMPLE CLEANING LIQUID |
| | S228 | AGITATION |
| | S229 | CENTRIFUGATION |
| | S230 | REMOVE SUPERNATANT |
| SECOND STAINING | S231 | DISPENSE ANTIBODY REAGENT |
| | S232 | AGITATION/REACTION |
| | S233 | DISPENSE SAMPLE CLEANING LIQUID |
| | S234 | AGITATION |
| | S235 | CENTRIFUGATION |
| | S236 | REMOVE SUPERNATANT |
| | S237 | DISPENSE SAMPLE CLEANING LIQUID |
| | S238 | AGITATION |
| | S239 | CENTRIFUGATION |
| | S240 | REMOVE SUPERNATANT |
| RETURNING CONTAINER | S241 | DISPENSE BUFFER SOLUTION |
| | S242 | AGITATION |
| | S243 | TRANSPORT CENTRIFUGE TUBE |

FIG. 5A

FIG. 5B

TRANSFER TO CENTRIFUGATION SECTION

FIG. 5C

TRANSFER TO CENTRIFUGATION SECTION

FIG. 6A

11    10a   20

21

P1    13   P2    10b
AGITATE SAMPLE    10c

FIG. 6B

11 DISPENSE SAMPLE    10a

13   P1     P2    10b
10c

FIG. 6C

11    10a

P1    13   P2    10b
AGITATE SAMPLE   10c

FIG. 6D

11 DISPENSE SAMPLE

10a
10b
10c

13   P1     P2

X1   X2
X

FIG. 7A DISPENSE ANTI-RED BLOOD CELL ANTIBODY

FIG. 7B DISPENSE ANTI-RED BLOOD CELL ANTIBODY

FIG. 7C DISPENSE SOLID PHASE

FIG. 7D DISPENSE SOLID PHASE

FIG. 7E MAGNETIZATION

FIG. 8A  DISPENSE SUPERNATANT

STAINING PROCESS IN CENTRIFUGATION SECTION

FIG. 8B  TRANSFER FROM CENTRIFUGATION SECTION

FIG. 8C  TRANSFER FROM CENTRIFUGATION SECTION

FIG. 8D

FIG. 9

EMBODIMENT AND
COMPARATIVE
EXAMPLE

%Teff

FIG. 10

EMBODIMENT AND
COMPARATIVE
EXAMPLE

%Treg

FIG. 11

EMBODIMENT (SEPARATION METHOD)

CD4 FLUORESCENT SIGNAL
INTENSITY

FIG. 12

COMPARATIVE EXAMPLE (HEMOLYSIS METHOD)

CD4 FLUORESCENT SIGNAL
INTENSITY

FIG. 13

EMBODIMENT (SEPARATION METHOD)

CD62L FLUORESCENT SIGNAL INTENSITY

FIG. 14

COMPARATIVE EXAMPLE (HEMOLYSIS METHOD)

CD62L FLUORESCENT SIGNAL INTENSITY

FIG. 15

EMBODIMENT (SEPARATION METHOD)

FOXP3 FLUORESCENT
SIGNAL INTENSITY

CD25 FLUORESCENT
SIGNAL INTENSITY

FIG. 16

COMPARATIVE EXAMPLE (HEMOLYSIS METHOD)

FOXP3 FLUORESCENT
SIGNAL INTENSITY

CD25 FLUORESCENT
SIGNAL INTENSITY

Europäisches
Patentamt

European
Patent Office

Office européen
des brevets

# EUROPEAN SEARCH REPORT

Application Number

EP 23 15 8499

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2014/073536 A1 (LIN PING [US] ET AL) 13 March 2014 (2014-03-13) <br> * paragraph [0002] * <br> * paragraph [0023] * <br> * paragraphs [0064] - [0070] * <br> * paragraph [0119] * <br> * paragraph [0134] * <br> * paragraph [0143] * <br> * paragraph [0243] * <br> * paragraph [0255] * <br> * paragraphs [0365] - [0367] * <br> * paragraphs [0396] - [0405] * <br> ----- | 1-20 | INV. <br> G01N1/30 <br> G01N1/31 |
| X | US 5 629 147 A (ASGARI MORTEZA [US] ET AL) 13 May 1997 (1997-05-13) <br> * page 4, lines 10-16 * <br> * page 9, lines 50-62 * <br> * page 12, lines 1-17 * <br> * example 1 * <br> ----- | 1,10 | |
| A | US 2021/302285 A1 (SHINYA RYUTARO [JP] ET AL) 30 September 2021 (2021-09-30) <br> * paragraphs [0078] - [0079] * <br> * paragraphs [0168] - [0169] * <br> * paragraphs [0338] - [0379] * <br> ----- | 1-20 | TECHNICAL FIELDS SEARCHED (IPC) <br><br> G01N |

The present search report has been drawn up for all claims

| Place of search | Date of completion of the search | Examiner |
|---|---|---|
| Munich | 21 June 2023 | Michalitsch, Richard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 15 8499

This annex lists the patent family members relating to the patent documents cited in the above-mentioned European search report.
The members are as contained in the European Patent Office EDP file on
The European Patent Office is in no way liable for these particulars which are merely given for the purpose of information.

21-06-2023

| Patent document cited in search report | | Publication date | Patent family member(s) | | Publication date |
|---|---|---|---|---|---|
| US 2014073536 | A1 | 13-03-2014 | US 2006252054 A1 | | 09-11-2006 |
| | | | US 2014073536 A1 | | 13-03-2014 |
| | | | US 2016040232 A1 | | 11-02-2016 |
| | | | US 2017089814 A1 | | 30-03-2017 |
| US 5629147 | A | 13-05-1997 | US 5629147 A | | 13-05-1997 |
| | | | US 5766843 A | | 16-06-1998 |
| | | | US 5858649 A | | 12-01-1999 |
| | | | US 5861253 A | | 19-01-1999 |
| US 2021302285 | A1 | 30-09-2021 | EP 3893002 A1 | | 13-10-2021 |
| | | | JP 2021162325 A | | 11-10-2021 |
| | | | US 2021302285 A1 | | 30-09-2021 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014528697 A

- JP 6170511 B